# EUROPEAN PATENT APPLICATION

(11) **EP 1 987 813 A1**
(43) Date of publication of application: **05.11.2008**
(21) Application number: 07107501.4
(22) Date of filing: 04.05.2007
(51) Int. Cl.: A61K 9/00, A61K 9/127, A61K 31/337

(54) **Injectable pharmaceutical formulation of taxoids in stable liposomes**

(71) Applicant: Pharmatex Italia Srl, 20121 Milano (IT)
(72) Inventor: De tomasso, Vincenzo, Dr., 20052, Monza (IT)
(74) Representative: Serravalle, Marco

(57) **Abstract**

The present invention concerns an injectable pharmaceutical composition of taxoids in stable liposomes. The formulation comprises the taxoid, a phospholipid, a viscosity improver and optionally a solvent for the phospholipid.

The formulation is prepared according to the following steps:
a) Solubilization of the phospholipid in a solvent, preferably ethanol;
b) Addition of a Taxoid and its solubilization under stirring;
c) Addition of a viscosity improver, preferably glycerol, under stirring;
d) The solution is sterilized by filtration with a PTFE hydrophobic membrane with a size of 0.22 micron, in sterile room (Class A);
e) In another vessel, sterile water for injection is prepared by filtration of water with a PTFE hydrophilic membrane with a size of 0.22 micron, in sterile room (Class A);
f) A minimal amount of water obtained in e) is added to the ethanol solution of d) to achieve the formation of liposomes. The suspension is left under vigorous stirring for at least 20'. The temperature is preferably comprised between 20 and 25°C;
g) The suspension is brought to the final volume by addition of sterile water obtained in e) at a pH comprised between 5.5 and 7.5;
h) The liposome's suspension is bottled sterilely.

## Description

The present invention relates to injectable pharmaceutical formulations of Taxoids, for example Paclitaxel or Docetaxel.

Taxoids are antitumor agents which are usually administered by injection, but due to their poor solubility in water it is difficult to prepare formulations which are acceptable from a therapeutic stand point.

At the present, commercially available formulations of Paclitaxel comprise a surfactant (Chromophor EL or Tween 80) and ethanol. Surfactants are very dangerous for the human body, since they produce side effects such as hypersensitivity. Furthermore, they produce the extraction of DEHP from plastic containers containing PVC. It is therefore necessary to carefully avoid the use of any plastic which contains PVC.

In the recent years many attempts have been made to prepare different formulations which do not contain surfactants. US 5,670,536 discloses pharmaceutical compositions comprising Docetaxel or a derivative thereof, at least one unsaturated phospholipid and at least a negative phospholipids different from the unsaturated phospholipid. The formulation consists of liposomes of very small size, usually lower than 100 nm. This formulation is preferably lyophilized or frozen for storing.

US 5,424,073 discloses liposomal-encapsulated taxol. These liposomes are obtained by combining a taxol, cardiolipin and a liposome-forming compound. The obtained liposomes are stable at room temperature for only 4 days.

Liposomes are spherical vesicles constituted by one or more phospholipids and optionally cholesterol. They form bilayers which normally contain a core of aqueous solution. Liposomes are classified according to the structure. Multilamellar liposomes are formed by several layers of phospholipids and have normally a size larger than 0.4 µm, and unilamellar liposomes, which are formed by a single bilayer. Unilamellar liposomes are in turn divided into large unilamellar liposomes, whose size is comprised between 0.05 and 0.20 µm and small unilamellar liposomes with size comprised between 0.02 and 0.05 µm.

The present invention relates to new formulations of taxoids, wherein the taxoid is present in multilamellar liposomes, generally having an average size by weight higher than 0.4 µm, preferably higher than 0.5 µm, most preferably comprised between 1 and 5 µm.

The liposomes of the present invention are stable for several months at room temperature and can be stored without requiring liophylization or freezing.

The present invention allows the preparation of liposome-containing injectable suspensions with a concentration equal to or higher than 6 mg/ml, stable in time and easy to dilute, as it is usually done in therapy, to a final concentration comprised between 0.3 mg/ml and 1.2 mg/ml.

The formulation according to the present invention is preferably prepared according to the following process:
a) Solubilization of the phospholipid in a solvent, preferably ethanol;
b) Addition of a taxoid and its solubilization under stirring;
c) Addition of viscosity improver under stirring;
d) The solution is sterilized by filtration with a PTFE hydrophobic membrane with a size of 0.22 micron, in sterile room (Class A).
e) In another vessel, sterile water for injection is prepared by filtration of water with a PTFE hydrophilic membrane with a size of 0.22 micron, in sterile room (Class A).
f) A minimal amount of water obtained in e) is added to the solution of d) to achieve the formation of liposomes. The suspension is left under vigorous stirring for at least 20'. The temperature is preferably comprised between 20 and 25°C.
g) The suspension is brought to the final volume by addition of sterile water obtained in e) at a pH comprised between 5.5 and 7.5
h) The liposome's suspension is bottled sterilely.

The phospholipid can be any commercially available phospholipid, for example, phosphatidyl choline, phosphatidyl serine, phosphatidyl inositol. Preferred phospholipid is phosphatidyl choline.

The amount of solvent used in step a) can vary in a broad range. The minimum amount corresponds to the amount needed for solubilization of the phospholipids. However, a larger amount can be used without prejudice. The maximum amount corresponds to the amount which does not allow formation of liposomes after addition of water. Preferred the concentration of solvent in the suspension of g) is comprised in the range of from 5 to 30 % by weight, preferably from 10 to 20 % by weight. However, it is also possible to remove the solvent after step d). In fact, the liposomes, once formed, are stable even without solvent in the suspension.

A viscosity improver is used in the preparation of the formulation. With viscosity improver it is intended a compound which is able to increase viscosity around the liposome. In this way, the liposome is more stable. Amongst the possible viscosity improver we can cite glycerol, propylene glycol and glycofurol. Glycerol is the preferred viscosity improver. The amount of viscosity improver can vary in a broad range, and preferably its concentration in the suspension of step g) can vary from 1 to 25% by weight, preferably from 1.5 to 15% by weight, most preferably from 2 to 10 % by weight. The viscosity improver can also be used as the solvent of step a). In this case, the preferred compound which can be used both as a solvent and as a viscosity improver is glycofurol.

The formulation according to the present invention presents several advantages in view of the formulations of the prior art:
1) Since liposomes are very stable in time, the formulation can be stored as such, without any need of lyophilizing or freezing it. This is also advantageous since these processes involve a stress on the liposomial membrane, which might be damaged as a result of it.
2) Absence of dangerous ingredients, e.g. surfactants.
3) Easiness of use for paramedicals.
4) Absence of interaction of the suspension with the plastic used for the intravenous administration.
5) Easiness of the production process, which does not require a specific apparatus, but it follows standard process procedures for sterile products.

### Examples

### Example 1

12 g of phosphatidyl choline (Epikuron 200^{™}) were solubilized in 12 g of ethanol at a temperature comprised between 20 and 25 °C, under mild stirring. After solubilization, 480 mg of Paclitaxel were added under mild stirring. Afterwards, 3.5 g of glycerol were added and the solution was left under stirring for 20'. The solution was sterilized by filtration with a PTFE hydrophobic membrane with a size of 0.22 micron, in sterile room (Class A). In another vessel, sterile water for injection was prepared by filtration of water with a PTFE hydrophilic membrane with a size of 0.22 micron, in sterile room (Class A). The ethanol solution of Paclitaxel was placed under vigorous stirring and 40 ml of sterile water were added and the suspension was left under stirring for 20' at a temperature comprised between 20 and 25 °C. The suspension is then brought to the final volume of 80 ml by addition of sterile water. The concentration of Paclitaxel was 6 mg/ml (which corresponds to the concentration of the commercially available Paclitaxel). The pH of the suspension was checked to verify that it was comprised between 5.5 and 7.5. The suspension was kept under stirring for 20' and was then sterilely bottled in vials of 5 ml, 16.7 ml, 25 ml and 50 ml.

### Example 2

12 g of phosphatidilcholine (Epikuron 200^{™}) were solubilized in 12 g of ethanol at a temperature comprised between 20 and 25 °C, under mild stirring. After solubilization, 480 mg of Paclitaxel were added under mild stirring. Afterwards, 3.5 g of glycerol were added and the solution was left under stirring for 20'. The solution was sterilized by filtration with a PTFE hydrophobic membrane with a size of 0.22 micron, in sterile room (Class A). In another vessel, sterile water for injection was prepared by filtration of water with a PTFE hydrophilic membrane with a size of 0.22 micron, in sterile room (Class A). The ethanol of the ethanol solution of Paclitaxel was removed by stripping under vacuum at a temperature of maximum 30 °C. The suspension was then brought to the final volume of 80 ml by addition of sterile water. The concentration of Paclitaxel was 6 mg/ml (which corresponds to the concentration of the commercially available Paclitaxel). The pH of the suspension was checked to verify that it was comprised between 5.5 and 7.5. The suspension was kept under stirring for 20' and was then sterilely bottled in vials of 5 ml, 16.7 ml, 25 ml and 50 ml.

### Example 3

The procedure of example 1 was repeated but the amount of Paclitaxel added was 200 mg and the final volume 166 ml. In this way, a concentration of 1.2 mg/ml of Paclitaxel in the liposome suspension was obtained.

The liposomes obtained by this method fully encapsulate the taxoid. This was evaluated analytically (by HPLC) on a suspension which was left to rest for 10 days, by performing the analysis on a sample taken from the top and on a sample from the bottom. Both samples showed the identical concentration of taxoid.

This test was also performed on samples kept for 30 and 60 days at 5 °C, 25 °C and 40 °C, and the results were always consistent: the concentration of taxoid at the top and at the bottom of the suspension was identical.

Furthermore, Table 1 and Table 2 report the size of liposomes obtained respectively in Example 1 and Example 3, immediately after formation (To) and after 1 and 2 months at 5 °C, at 25 °C and at 40 °C. The size of liposomes was measured by using a MALVERN Master Sizer 2000. The percent is expressed as % by weight.

### PARTICLES DISTRIBUTION

**Table 1**

| **%** | **T₀** | **After 1 month** | | | **After 2 months** | | |
|---|---|---|---|---|---|---|---|
| | | **5°C** | **25°C** | **40°C** | **5°C** | **25°C** | **40°C** |
| 90 | 22.8 µm | 22.51 µm | 22.12 µm | 22.72 µm | 23.39 µm | 24.1 µm | 24.47 µm |
| 50 | 3.16 µm | 2.88 µm | 2.67 µm | 2.91 µm | 2.54 µm | 3.02 µm | 3.16 µm |
| 10 | 0.66 µm | 0.71 µm | 0.836 µm | 0.78 µm | 0.84 µm | 0.87 µm | 0.90 µm |

**Table 2**

| **%** | **T₀** | **After 1 month** | | | **After 2 months** | | |
|---|---|---|---|---|---|---|---|
| | | **5°C** | **25°C** | **40°C** | **5°C** | **25°C** | **40°C** |
| 90 | 6.63 µm | 6.40 µm | 6.92 µm | 7.18 µm | 6.47 µm | 6.67 µm | 7.56 µm |
| 50 | 2.15 µm | 2.06 µm | 2.22 µm | 2.13 µm | 2.16 µm | 2.22 µm | 2.06 µm |
| 10 | 0.71 µm | 0.75 µm | 0.77 µm | 0.78 µm | 0.76 µm | 0.77 µm | 0.75 µm |

Another method for the control of the amount of taxoid encapsulated in liposomes is through filtration of the homogeneous liposome suspension with a 0.45 micron hydrophilic membrane. The filtrate was analyzed and the amount of taxoid measured was 0.6 % of the total amount dissolved. This amount corresponds to the taxoid which remains dissolved in water due to the presence of ethanol.

## Claims

1. Pharmaceutical formulation of taxoids **characterized in that** the formulation comprises multilamellar liposomes.

2. Formulation according to claim 1 wherein the taxoid is selected from Paclitaxel and Docetaxel.

3. Formulation according to claims 1-2 wherein the liposomes have an average size by weight higher than 0.4 µm, preferably higher than 0.5 µm, most preferably comprised between 1 and 5 µm.

4. Formulation according to any of the preceding claims wherein the formulation comprises a taxoid, a phospholipid, a viscosity improver and optionally a solvent.

5. Formulation according to claim 4 wherein the phospholipid is phosphatidyl choline.

6. Formulation according to claims 1-5 wherein the formulation does not contain cholesterol.

7. Formulation according to claims 1-6 wherein the solvent is ethanol and it is present in an amount comprised between 5 and 30 % by weight.

8. Formulation according to claims 1-7 wherein viscosity improver is glycerol and it is present in an amount comprised between 1 and 25 % by weight.

9. Process for the preparation of a pharmaceutical formulation of a taxoid, according to the following process:
a) Solubilization of a phospholipid in a solvent, preferably ethanol;
b) Addition of a Taxoid and its solubilization under stirring;
c) Addition of a viscosity improver, preferably glycerol, under stirring;
d) The solution is sterilized by filtration with a PTFE hydrophobic membrane with a size of 0.22 micron, in sterile room (Class A);
e) In another vessel, sterile water for injection is prepared by filtration of water with a PTFE hydrophilic membrane with a size of 0.22 micron, in sterile room (Class A);
f) A minimal amount of water obtained in e) is added to the solution of d) to achieve the formation of liposomes; the suspension is left under vigorous stirring for at least 20'; the temperature is preferably comprised between 20 and 25°C;
g) The suspension is brought to the final volume by addition of sterile water obtained in e) at a pH comprised between 5.5 and 7.5;
h) The liposome's suspension is bottled sterilely.
